# EUROPÄISCHE PATENTANMELDUNG

(11) **EP 4 140 509 A1**
(43) Veröffentlichungstag der Anmeldung: **01.03.2023**
(21) Anmeldenummer: 21192866.8
(22) Anmeldetag: 24.08.2021
(51) Int. Cl.: A61L 9/14, B01D 53/72, F24F 3/16, A61L 2/22

(54) **VORRICHTUNG UND VERFAHREN ZUR LUFTREINIGUNG UND/ODER -DESINFEKTION**

(71) Anmelder: Dr. Langhein GmbH, 16321 Börnicke bei Bernau (DE)
(72) Erfinder: Dr. Langhein, Siegfried, 18195 Cammin (DE)
(74) Vertreter: ETL IP Patent- und Rechtsanwaltsgesellschaft mbH

(57) **Zusammenfassung**

Vorrichtung und Verfahren zur Luftreinigung und/oder - desinfektion von in einem Luftkanal (1) strömender Luft von Bakterien und/oder Viren und/oder anderweitigen Keimen und/oder Partikel, wobei mindestens ein Teilluftstrom bypassartig in ein Gehäuse (2) mit einem darin angeordneten Luftbehandlungsraum umgelenkt wird, im Luftbehandlungsraum sich eine chemische Substanz in gasförmigen oder vernebelten Zustand mit freien Radikalen befindet, so dass ein Gemisch gebildet wird in dem die Bakterien und/oder Viren und/oder anderweitigen Keime und/oder Partikel durch in Kontakt treten mit und/oder Andocken der freien Radikale unschädlich/unwirksam gemacht werden, und die so behandelte Luft in den Luftkanal (1) zurückgeführt und durch einen Verteiler (8) verteilt wird.

## Beschreibung

Die Erfindung betrifft die Reinigung und/oder Desinfektion von mit Bakterien und/oder Viren und/oder anderweitigen Keimen und/oder Partikel belasteter Luft.

Insbesondere mit dem Auftreten belasteter Luft durch den Virus SARS-Cov-2 (auslösend für die Covid-19-Symptomiken und/oder "Long Covid") und seinen fortwährenden Mutationen, wurden spätestens seit Anfang 2020 weltweit zahlreiche Schutzmaßnahmen gegen das Erkranken der Menschen in nahezu allen Ländern der Welt in Gang gesetzt und ergriffen - pandemiebedingt. Dabei fanden u. a. auch Lösungen Verwendung, die bereits seit längerem Stand der Technik sind.

Generell hat die Pandemie mit dem Coronavirus gezeigt, dass mitunter die größten Ansteckungsherde geschlossene und wenig durchlüftete Räume darstellen. Provisorische Wege, wie das zyklische Öffnen von Fenstern, sind besonders in der kalten Jahreszeit nur bedingt geeignet zum Luftaustausch; bspw. in Kita- und Schuleinrichtungen, Krankenhausräumen, wie OPs, in Behörden, in Konzertsälen und dergleichen.

Entwickelt wurden deshalb Lüftungs- und Luftbefeuchtungsgeräte, die zumeist in entsprechenden Räumen separat oder gebündelt aufgestellt werden. Dazu gehört auch die Vorrichtung und das Verfahren zum Filtern und Befeuchten von Luft, die Gegenstand des erteilten Patentes DE 10 2007 017 750 B1 sind.

Der größte Teil der gegenwärtig hergestellten Luftreinigungsgeräte sind in Räumen aufgestellte Standgeräte mit Umluft bzw. Luftaustausch unter Verwendung von HEPA-Filtern H13, H14, die eine Durchlässigkeit von < 1 µm (PM₂) aufweisen (*National Air Quality Standard for Particulate*). Diese Filter können zwar auch Viren und/oder Bakterien herausfiltern, diese werden aber typischerweise dabei nicht komplett unwirksam gemacht, sondern eben nur herausgefiltert.

Des Weiteren gibt es Vorschläge zur Verwendung von UVC-Strahlung, die jedoch nicht mit der menschlichen Haut in Verbindung kommen darf; was damit ein Gefahrenpotenzial darstellt.

Bekannt sind weiter Luftfilter in modularer Bauweise, die sowohl eine rein mechanische Trennwirkung aufweisen als auch eine chemische und/oder antimikrobielle Wirkung gegen nanodisperse Luftverunreinigungen besitzen können. Bei derartigen Filtern werden Desinfektionslösungen auf Basis von Wasserstoffperoxid (H₂O₂) eingesetzt. Diese haben den Nachteil, dass Wasserstoffperoxid in die Luft freigesetzt wird und die Dämpfe, je nach Konzentration, ätzend auf die Haut und/oder Schleimhäute eines Menschen wirken können.

Im Stand der Technik wird deshalb zur Desinfektion von Luft vorgeschlagen, die Luft mit Wasserstoffperoxid anzureichern und eine katalytische Zersetzung des Wasserstoffperoxids vorzunehmen, um u. a. OH-Radikale herauszulösen, die wiederum innerhalb des Katalysators auf das Wasserstoffperoxid-Luftgemisch einwirken, so dass einerseits deren anti-bakterielle und/oder anti-virale Wirkung auf den Luftstrom ausnutzbar ist, und andererseits durch diese in Form einer Kettenreaktion weitere Radikale gebildet werden; bspw. freie OH- und O-Radikale. Ein Reinluftstrom soll den Katalysator verlassen, wobei das Wasserstoffperoxid zu Wasser und Sauerstoff umgesetzt ist (siehe bspw. EP 0 970 706 A1**).**

Nachteilig bei derartigen Standgeräten ist das Preis-Leistungsverhältnis; denn eines dieser letztgenannten Geräte kann nur die Luft eines Raumes behandeln.

Mithin ist es eine Aufgabe der Erfindung, mittels einer Vorrichtung und eines Verfahrens die Luft einer Vielzahl von Räumen gleichzeitig zu reinigen und zu desinfizieren, wobei die Bakterien und/oder Viren und/oder anderweitige Keime und/oder Partikel unwirksam gemacht werden sollen; also bspw. nicht mehr krankheitsauslösend sind - insbesondere gegenüber Covid-19.

Gelöst wird diese Aufgabe mit den Merkmalen der Ansprüche 1 und 15. Vorteilhafte Ausgestaltungen sind Gegenstand der Unteransprüche.

Die erfindungsgemäße Vorrichtung zur Luftreinigung von in einem Luftkanal strömender Luft umfasst mindestens ein Gehäuse mit einem darin angeordneten Luftbehandlungsraum, der über mindestens zwei Anschlüsse zum Luftkanal verfügt, so dass Luft bypassartig aus dem Luftkanal in den Luftbehandlungsraum überströmt, im Luftbehandlungsraum sich eine chemische Substanz in gasförmigen und/oder vernebelten Zustand mit freien Radikalen befindet, so dass ein Gemisch bildbar ist, das über einen Verteiler in den Luftstrom zurückführbar ist, wobei der Luftkanal der einer Klimaanlage und/oder einer Belüftungseinrichtung ist, und der Verteiler für die behandelte Luft im Luftkanal in Strömungsrichtung vor dem ersten Auslass in die zu klimatisierenden und/oder zu belüftenden Räume angeordnet ist.

Als freie Radikale bildende chemische Substanz ist in erster Linie Wasserstoffperoxid (H₂O₂) in wässriger Lösung erfindungsgemäß vorgesehen, da dies vorrichtungs- und verfahrensbedingt eben gerade nicht (mehr) physiologisch toxisch auf einen Menschen wirkt. Dieses wird durch eine Phosphorsäure in niedriger Konzentration stabilisiert (Einsatz unter 1 g einer Phosphorsäure in zumeist handelsüblicher 10%igen Wasserlösung, bevorzugt 0,5 g, mehr bevorzugt unter 0,5 g), bevorzugt also in einer Konzentration von 1 g bis 0,01 g Phosphorsäure in bspw. 10%iger Wasserlösung, vorzugsweise von 0,5 g bis 0,05 g Phosphorsäure in bspw. 10%iger Wasserlösung. Das Wasserstoffperoxid selbst wird in erster Linie in einer Konzentration von 1 bis 30%, vorzugsweise von 3 bis 25%, mehr bevorzugt von 5 bis 15%, am meisten bevorzugt in einer Konzentration von 5 bis 8 % eingesetzt.
Bei dieser Behandlung wird bereits ein Großteil der Viren und/oder Bakterien und/oder anderweitiger Keime und/oder Partikel unwirksam gemacht, nämlich dahingehend, dass das Einwirken der freiwerdenden OH- und O-Radikale auf ein Virus (anti-viral), Bakterium (anti-bakteriell), Keim, Partikel, diese mindestens in ihrem Andockprozess an eine menschliche Zelle unwirksam machen.

Gegebenenfalls verbleibendes Wasserstoffperoxid und/oder Restviren verlassen, wenn nicht weitere Maßnahmen im Behandlungsraum erfolgen, diesen und gelangen zu einem Verteiler für die behandelte Luft im Luftkanal.

Dieser Verteiler ist bevorzugt ein Rohrgitter im Luftkanal, wobei die Rohre des Rohrgitters Öffnungen in Form von Bohrungen bzw. Schlitzen gerichtet in Strömungsrichtung aufweisen. Das dient in erster Linie der Beaufschlagung des ganzen Luftkanalquerschnitts mit behandelter Luft.

Der Verteiler, bevorzugt aus Platin oder einer Platinlegierung, übernimmt dabei noch eine weitere Funktion. Durch die starke Oxidationswirkung von Platin oder einer Platinlegierung werden die ggf. verbliebenen Viren und H₂O₂-Anteile weiter unwirksam gemacht bzw. werden sie an dem Verteiler gebunden.

Um sicher vollständig viren- und bakterienfreie Luft zu erhalten, wird somit ein zweistufiges Verfahren erfindungsgemäß realisiert.

Wird nicht der ganze Luftstrom einer Behandlung unterzogen, sondern nur ein Teilluftstrom aus dem Luftkanal zur Behandlung umgeleitet, ist dieser bevorzugt so bemessen, dass die den Verteiler verlassende Luft ausreichend ist, um den vollen Luftstrom ansteckungsresistent zu machen. Dabei muss der Verteiler nicht aus Platin oder einer Platinlegierung bestehen.
Diese Verfahrensweise kann auch praktiziert werden, wenn der gesamte Luftstrom eine Behandlung erfährt.

Alle Mittel zur Luftreinigung und Luftdesinfektion sind bevorzugt in einer baulichen Einheit im Gehäuse zusammengeführt, wobei aus dem Gehäuse die Luftanschlüsse herausragen und das Gehäuse über Befestigungen für die Montage und Abdichtung gegenüber dem Luftkanal verfügt.

Eine derartige Vorrichtung hat große Vorteile. Sie ist auf die Raumgrößen anpassbar/skalierbar und kann gleichzeitig für mehrere Räume, sogar für eine Vielzahl an Räumen, genutzt werden, je nach Anordnung der Belüftungs-/Klimaanlage. Denkbar ist bspw., dass eine gesamte Kita, Schule, Behörde oder ein gesamtes Krankenhaus mittels einer geeigneten Vorrichtung der Erfindung mit gereinigter/desinfizierter Luft versorgt wird, wenn die Vorrichtung in den Lüftungs- und/oder Klimaanlagenkanal an einer zentralen geeigneten Auslassstelle montiert wird, bevor die Luft der zentralen Auslassstelle daraufhin in die einzelnen Räume verteilt wird.

Weiterhin kann die Vorrichtung Projektbestandteil einer Klimaanlage bzw. Belüftungsanlage sein oder zur Nachrüstung genutzt werden. Zum Nachrüsten wird der Luftkanal geöffnet und ein für die Vorrichtung geeigneter Abschnitt entfernt. Es bedarf dann lediglich noch der Verbindung von Anschlüssen des Luftkanals mit den Befestigungsmitteln des Gehäuses sowie die Abdichtung der Verbindung.

Der Überwachungs- und Betriebsaufwand einer derartigen Anlage sind gering. Es bedarf lediglich der Signalisierung des Füllstandes der Lösung(en) und in größeren Abständen eine Kontrolle der Anschlüsse zum Luftkanal und des Verteilers. Auch ein geeignetes wieder Auffüllen der Reaktanten und Komponenten ist angezeigt; insbesondere der wässrigen Wasserstoffperoxidlösung und/oder der Phosphorsäure.

Eine weitere vorteilhafte Ausgestaltung sieht vor, dass die an das Becken abgegebene Menge an freie Radikale bildende bzw. enthaltende Flüssigkeiten bzw. Gase steuer- bzw. regelbar ist.

Mit dieser und weiterer Ausgestaltungen werden Voraussetzungen dafür geschaffen, dass die Vorrichtung nur ein paar Modelle benötigt. "Zwischengrößen" lassen sich durch die Steuer- und Regeltechnik einstellen. Ebenso ist auf diese Weise eine Reaktion auf den Grad der Luftverschmutzung mit Viren und/oder Bakterien und/oder anderweitigen Keimen und/oder Partikel möglich.

Bei einer bevorzugten Ausführung sind weiter im Luftkanal eine oder mehrere Anschlüsse zum Einströmen von Luft vorgesehen, wobei diese vorzugsweise eine trichterförmige Öffnung entgegen der Strömungsrichtung der Luft gerichtet aufweisen. Das kann zur Erhöhung des Durchsatzes führen, gewährleistet eine Luftentnahme gleichmäßig über den Luftkanalquerschnitt und ist parallel mit der Steuerung der freie Radikale enthaltenden bzw. erzeugenden Lösung zu handhaben.

Werden zum Vernebeln Erreger auf piezoelektrischer Basis eingesetzt, wird in vorteilhafter Weise dem Verteiler in Strömungsrichtung ein Faraday'scher Käfig nachgeordnet, so dass elektronische Einrichtungen im Umfeld nicht beeinträchtigt werden.

Verfahrensseitig sieht die Erfindung vor, dass zur Luftreinigung von in einem Luftkanal strömender Luft von Bakterien und/oder Viren und/oder anderweitiger Keime und/oder Partikel mindestens ein Teilluftstrom bypassartig in ein Gehäuse mit einem darin angeordneten Luftbehandlungsraum umgelenkt wird, im Luftbehandlungsraum sich eine chemische Substanz in gasförmigen und/oder vernebelten Zustand mit freien Radikalen befindet, so dass ein Gemisch gebildet wird in dem die Bakterien und/oder Viren und/oder anderweitigen Keime und/oder Partikel durch Andocken unschädlich gemacht werden und die so behandelte Luft in den Luftkanal zurückgeführt und durch einen Verteiler verteilt wird. Vorteilhaft ist, wenn Wasserstoffperoxid in wässriger Lösung als chemische Substanz mit freien Radikalen eingesetzt wird und das Wasserstoffperoxid eine stabilisierende Phosphorsäure enthält.

Die Konzentration des Wasserstoffperoxids beträgt 1 bis 30%, vorzugsweise von 3 bis 25%, mehr bevorzugt von 5 bis 15%, und am meisten bevorzugt von 5 bis 8%.

Die stabilisierende Phosphorsäure wird in einer Konzentration von vorzugsweise 0,05 bis 0,5 g in einer bspw. 10%igen Wasserlösung eingesetzt. Der Fachmann erkennt, dass mithin bspw. für eine 30%ige Wasserstoffperoxidlösung 0,5 g Phosphorsäure in 10%iger Wasserlösung für die Stabilisierung einzusetzen wäre. Entsprechend wäre im Falle einer 3%igen Wasserstoffperoxidlösung (als um das 10-fache kleiner) auch nur eine 0,05 g Phosphorsäure in 10%iger Lösung einzusetzen. Dies ist nur beispielhaft, und der Fachmann kann mit Standardmethoden den jeweiligen tatsächlichen Bedarf an Reaktanten ausrechnen und den Gegebenheiten entsprechend nach oben oder unten skalieren.

Der Verteiler besteht bevorzugt aus Platin oder einer Platinlegierung, um Reste von Bakterien und/oder Viren und/oder anderweitigen Keimen und/oder Partikel aus der aus dem Luftbehandlungsraum zurück in den Luftkanal geführten Luft unschädlich/unwirksam zu machen.

Natürlich ist es auch möglich, dass der zur Behandlung umgeleitete Teilluftstrom aus dem Luftkanal so bemessen ist, dass die den Verteiler verlassende Luft ausreichend ist, um den vollen Luftstrom ansteckungsresistent zu machen. Das gilt auch, wenn der gesamte Luftstrom eine Behandlung erfährt.

Deshalb sieht eine vorteilhafte Ausgestaltung auch vor, dass die in den Luftbehandlungsraum abgegebenen Mengen an freie Radikale enthaltenden oder bildenden flüssigen oder gasförmigen chemischen Substanzen gesteuert- bzw. geregelt werden.

Die freie Radikale enthaltenden oder bildenden chemischen Substanzen können im Luftbehandlungsraum mittels Piezoelementen auf einfache Weise vernebelt werden, wobei die durch die Piezoelemente erzeugten Schwingungen nach außen durch einen Faraday'sehen Käfig kompensiert werden.

Die Erfindung soll ferner anhand der Zeichnung erläutert werden.

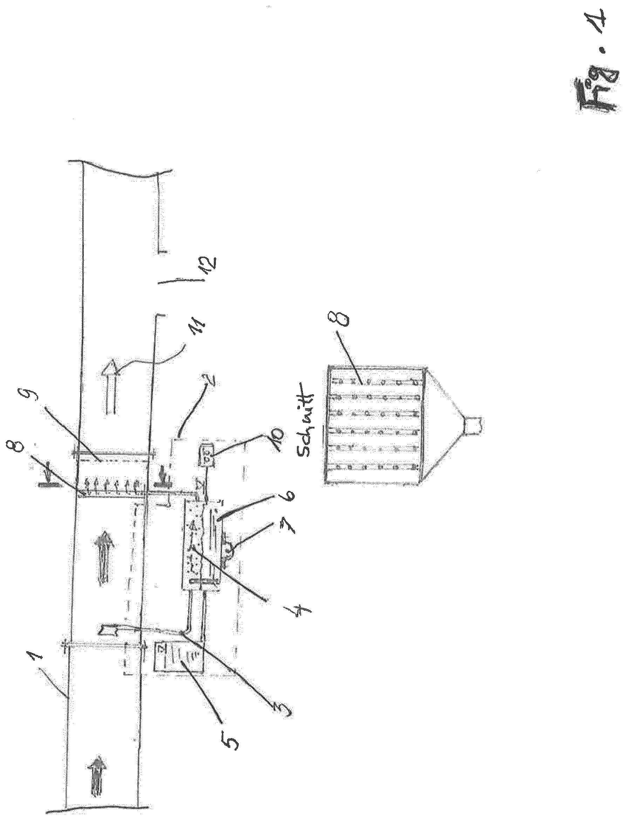

Die Vorrichtung in **Fig. 1** verfügt über alle Mittel zur Luftreinigung und/oder Luftdesinfektion, die in einer baulichen Einheit umgeben von einem Gehäuse 2 zusammengeführt sind, wobei aus dem Gehäuse 2 die Luftanschlüsse herausragen und das Gehäuse 2 über Befestigungen für die Montage und Abdichtung gegenüber dem Luftkanal 1 einer RLT-Anlage (raumlufttechnische Anlage) verfügen.

Der Luftkanal 1 ist aus Segmenten in Strömungsrichtung 11 zusammengesetzt, wobei die einzelnen Segmente gegeneinander abgedichtet sind. Das entspricht vom Aufbau her dem heute üblichen Stand der Technik.

Die mit dem Luftkanal verbundene Vorrichtung zur Luftreinigung und/oder Luftdesinfektion umfasst die im Gehäuse 1 angeordneten Teile: eine Mischeinrichtung mit Mischkammer 4, einen mit H₂O₂ gefüllten Behälter 5, Piezoelemente 7, die im Hochfrequenzbereich im Wellenbereich bei bspw. 300 bis 500 Mikrometer arbeiten und das H₂O₂ Gemisch vernebeln. Dem Fachmann leuchtet dabei ein, dass der Hochfrequenzbereich im Wellenbereich stets den Gegebenheiten entsprechend geeignet gewählt, und damit skaliert werden kann. Der Bereich von 300 bis 500 Mikrometer ist mithin rein beispielhaft zu verstehen und kann größer aber auch geringen ausfallen in Abhängigkeit der Gegebenheiten.

Die zu reinigende und/oder zu desinfizierende Luft wird über mindestens einen - vorzugsweise mehrere - Anschlüsse zum Luftstrom 3 diesem in einer berechneten Menge entnommen und gelangt in die Mischeinrichtung 4, wo eine Mischung mit dem Aerosolnebel erfolgt. Dabei kommen freie Radikale (bevorzugt OH- und O-Radikale), entstanden aus dem vernebelten H₂O₂ Gemisch, mit den Viren und/oder Bakterien und/oder anderweitigen Keimen und/oder Partikel in direkten Kontakt, und diese werden mithin stark reaktiv unwirksam gemacht. Das ist ein wesentlicher Vorteil gegenüber HEPA-Filtern.

Die so gereinigte und/oder desinfizierte Luft wird über einen Verteiler 8, hier ein Rohrgitter, dem Luftstrom zugeführt. Die Austrittsöffnungen aus dem Verteiler-Rohr-Gitter sind Bohrungen und/oder Schlitze, gerichtet jeweils in Strömungsrichtung 11.

Das Verteiler-Rohr-Gitter ist aus Platin, das durch die starke Oxidationswirkung bewirkt, dass die den Behandlungsraum verlassenden verbliebenen Viren und H₂O₂-Anteile unwirksam gemacht bzw. gebunden werden.

Da die Vernebelung durch die Piezoelemente 7 im Hochfrequenzbereich erfolgt, ist es vorteilhaft, wenn nachfolgend nach dem Rohrgitter 8 ein Faraday'scher Käfig 9 im Luftkanal 1 angeordnet ist. Er schützt u. a. IT-Geräte, medizinische Geräte etc. vor Störungen bzw. Ausfall.

Die Vorrichtung selbst ist zweckmäßiger Weise mit einer Reihe von Steuerungs-/Regelungselementen ausgerüstet, um für eine optimale Luftmenge zu sorgen, das Aerosol optimal einzusetzen, Ausfälle, z.B. bedingt durch einen leeren Aerosolbehälter, den CO₂-Gehalt der Luft vor der Vorrichtung zu prüfen und mit einer erhöhten Luftmenge zu arbeiten und/oder die Aerosolmenge zu verändern. Dazu dient z.B. auch ein Schwimmerschalter 10 für die im Becken 6 befindliche Flüssigkeit.

Die Erfindung ist universell einsetzbar, d.h. sie kann in öffentlichen Räumen genauso genutzt werden wie im Eigenheim, in Werkstätten oder Montagehallen oder auch in Viehställen. Der Fachmann erkennt, dass es mannigfaltige Anwendungsformen der Erfindung gibt, und die zuvor genannten nicht als limitierend aufzufassen sind.

### Bezugszeichenliste

- 1: Luftkanal einer Klimaanlage bestehend aus verschraubten Segmenten
- 2: Gehäuse mit apparativer Ausrüstung
- 3: Anschlüsse zum Luftkanal (Entnahmerohr)
- 4: Mischeinrichtung mit Mischkammer
- 5: mit H₂O₂ gefüllter Behälter
- 6: Becken
- 7: Piezoelement
- 8: Verteiler (Rohr-Gitter)
- 9: Faraday'scher Käfig
- 10: Schwimmerschalter
- 11: Strömungsrichtung
- 12: Auslass im Raum

Mit dem obigen Kontext der Gesamtoffenbarung, umfasst die vorliegende Erfindung ferner die im folgenden nummerierten Ausführungsformen:
1. Vorrichtung zur Luftreinigung und/oder -desinfektion von in einem Luftkanal (1) strömender Luft umfassend mindestens ein Gehäuse (2) mit einem darin angeordneten Luftbehandlungsraum, der über mindestens zwei Anschlüsse (3) zum Luftkanal (1) verfügt, so dass Luft bypassartig aus dem Luftkanal (1) in den Luftbehandlungsraum überströmt, im Luftbehandlungsraum sich eine chemische Substanz in gasförmigen oder vernebelten Zustand mit freien Radikalen befindet, so dass ein Gemisch bildbar ist, wobei das Gemisch über einen Verteiler (8) in den Luftstrom zurückführbar ist, **dadurch gekennzeichnet, dass**
   der Luftkanal (1) der einer Klimaanlage und/oder einer Belüftungseinrichtung ist und der Verteiler (8) für die behandelte Luft im Luftkanal (1) in Strömungsrichtung (11) vor dem ersten Auslass (12) in die zu klimatisierenden und/oder zu belüftenden Räume angeordnet ist.
2. Vorrichtung nach Ausführungsform 1, **dadurch gekennzeichnet, dass**
   das Becken (6) Wasserstoffperoxid in wässriger Lösung (5) als chemische Substanz, bevorzugt mit freien Radikalen, mehr bevorzugt mit OH- und/oder O-Radikalen enthält.
3. Vorrichtung nach Ausführungsform 2, **dadurch gekennzeichnet, dass**
   die Konzentration des Wasserstoffperoxids von 1 bis 30%, bevorzugt 3 bis 25%, mehr bevorzugt von 5 bis 15%, und am meisten bevorzugt 5 bis 8% beträgt.
4. Vorrichtung nach einer der Ausführungsformen 1 bis 3, **dadurch gekennzeichnet, dass**
   die chemische Substanz, bevorzugt mit den freien Radikalen, eine stabilisierende Phosphorsäure enthält, vorzugsweise in einer Konzentration von 0,01 bis 0,5 g in wässriger Lösung, mehr bevorzugt in einer Konzentration von 0,05 bis 0,5 g in wässriger Lösung.
5. Vorrichtung nach einer der Ausführungsformen 1 bis 4, **dadurch gekennzeichnet, dass**
   der Verteiler (8) aus Platin oder einer Platinlegierung besteht.
6. Vorrichtung nach einer der Ausführungsformen 1 bis 5, **dadurch gekennzeichnet, dass**
   der zur Behandlung umgeleitete Luftstrom aus dem Luftkanal (1) ein Teilluftstrom ist, der so bemessen ist, dass die den Verteiler (8) verlassende Luft ausreichend ist, um den vollen Luftstrom ansteckungsresistent zu machen.
7. Vorrichtung nach einer der Ausführungsformen 1 bis 5, **dadurch gekennzeichnet, dass**
   der gesamte Luftstrom eine Behandlung erfährt, wobei die den Verteiler (8) verlassende Luft ansteckungsresistent ist.
8. Vorrichtung nach einer der Ausführungsformen 1 bis 7, **dadurch gekennzeichnet, dass**
   die in den Behandlungsraum abgegebenen Mengen an freie Radikale enthaltenden oder bildenden flüssigen oder gasförmigen chemischen Substanzen steuer- bzw. regelbar ist.
9. Vorrichtung nach einer der Ausführungsformen 1 bis 8, **dadurch gekennzeichnet, dass**
   zum Vernebeln der freie Radikale enthaltenden oder bildenden chemischen Substanzen Piezoelemente (7) eingesetzt sind.
10.Vorrichtung nach einer der Ausführungsformen 1 bis 9, **dadurch gekennzeichnet, dass**
   eine oder mehrere Anschlüsse zum Einströmen von Luft bestehen, die vorzugsweise eine trichterförmige Öffnung entgegen der Strömungsrichtung (12) der Luft aufweisen.
11.Vorrichtung nach einer der Ausführungsformen 1 bis 10, **dadurch gekennzeichnet, dass**
   die Zurückführung der behandelten Luft über den als Rohrgitter im Luftkanal (1) ausgebildeten Verteiler (8) erfolgt, wobei die Rohre des Rohrgitters Öffnungen in Form von Bohrungen bzw. Schlitzen gerichtet in Strömungsrichtung (12) aufweisen.
12.Vorrichtung nach Ausführungsform 11, **dadurch gekennzeichnet, dass**
   die Rohre des Rohrgitters im montierten Zustand senkrecht oder nahezu senkrecht angeordnet sind.
13.Vorrichtung nach einer der Ausführungsformen 9 bis 10, **dadurch gekennzeichnet, dass**
   bei Verneblern auf piezoelektrischer Grundlage dem Verteiler (8) in Strömungsrichtung (11) ein Faraday'scher Käfig (9) nachgeordnet ist.
14.Vorrichtung nach einer der Ausführungsformen 1 bis 11, **dadurch gekennzeichnet, dass**
   alle Mittel zur Luftreinigung und/oder Luftdesinfektion in einer baulichen Einheit eines Gehäuses (2) zusammengeführt sind, wobei aus dem Gehäuse (2) die Luftanschlüsse herausragen und das Gehäuse (2) über Befestigungsmittel für die Montage und Abdichtung gegenüber dem Luftkanal (1) verfügt.
15.Verfahren zur Luftreinigung von in einem Luftkanal (1) strömender Luft von Bakterien und/oder Viren und/oder anderweitigen Keimen und/oder Partikel, wobei mindestens ein Teilluftstrom bypassartig in ein Gehäuse (2) mit einem darin angeordneten Luftbehandlungsraum umgelenkt wird, im Luftbehandlungsraum sich eine chemische Substanz in gasförmigen oder vernebelten Zustand mit freien Radikalen befindet, so dass ein Gemisch gebildet wird in dem die Bakterien und/oder Viren und/oder anderweitigen Keimen und/oder Partikel durch in Kontakt treten mit und/oder Andocken der freien Radikale unschädlich/unwirksam gemacht werden, und die so behandelte Luft in den Luftkanal (1) zurückgeführt und durch einen Verteiler (8) verteilt wird.
16.Verfahren nach Ausführungsform 15, **dadurch gekennzeichnet, dass**
   Wasserstoffperoxid in wässriger Lösung (5) als chemische Substanz mit freien Radikalen eingesetzt wird und das Wasserstoffperoxid eine stabilisierende Phosphorsäure enthält.
17.Verfahren nach Ausführungsform 16, **dadurch gekennzeichnet, dass**
   die Konzentration des Wasserstoffperoxids von 1 bis 30%, bevorzugt 3 bis 25%, mehr bevorzugt von 5 bis 15%, und am meisten bevorzugt 5 bis 8% beträgt.
18.Verfahren nach Ausführungsform 16 oder 17, **dadurch gekennzeichnet, dass**
   die chemische Substanz, bevorzugt mit den freien Radikalen, eine stabilisierende Phosphorsäure enthält, vorzugsweise in einer Konzentration von 0,01 bis 0,5 g in wässriger Lösung, mehr bevorzugt in einer Konzentration von 0,05 bis 0,5 g in wässriger Lösung.
19.Verfahren nach einer der Ausführungsformen 15 bis 18, **dadurch gekennzeichnet, dass**
   der Verteiler (8) aus Platin oder einer Platinlegierung besteht, um Reste von Bakterien und/oder Viren und/oder anderweitigen Keimen und/oder Partikel aus der aus dem Luftbehandlungsraum zurück in den Luftkanal (1) geführten Luft unschädlich/unwirksam zu machen.
20.Verfahren nach einer der Ausführungsformen 15 bis 18, **dadurch gekennzeichnet, dass**
   der zur Behandlung umgeleitete Teilluftstrom aus dem Luftkanal (1) so bemessen ist, dass die den Verteiler (8) verlassende Luft ausreichend ist, um den vollen Luftstrom ansteckungsresistent zu machen.
21.Verfahren nach einer der Ausführungsformen 15 bis 18, **dadurch gekennzeichnet, dass**
   der gesamte Luftstrom eine Behandlung erfährt, wobei die den Verteiler (8) verlassende Luft ansteckungsresistent ist.
22.Verfahren nach einer der Ausführungsformen 15 bis 21, **dadurch gekennzeichnet, dass**
   die in den Luftbehandlungsraum abgegebenen Mengen an freie Radikale enthaltenden oder bildenden flüssigen oder gasförmigen chemischen Substanzen gesteuert- bzw. geregelt werden.
23.Verfahren nach einer der Ausführungsformen 15 bis 22, **dadurch gekennzeichnet, dass**
   die freie Radikale enthaltenden oder bildenden chemischen Substanzen im Luftbehandlungsraum mittels Piezoelementen (7) vernebelt werden.
24.Verfahren nach Ausführungsform 23, **dadurch gekennzeichnet, dass**
   die durch die Piezoelemente (7) erzeugten Schwingungen nach außen durch einen Faraday'sehen Käfig kompensiert werden.

## Patentansprüche

1. Vorrichtung zur Luftreinigung und/oder -desinfektion von in einem Luftkanal (1) strömender Luft umfassend mindestens ein Gehäuse (2) mit einem darin angeordneten Luftbehandlungsraum, der über mindestens zwei Anschlüsse (3) zum Luftkanal (1) verfügt, so dass Luft bypassartig aus dem Luftkanal (1) in den Luftbehandlungsraum überströmt, im Luftbehandlungsraum sich eine chemische Substanz in gasförmigen oder vernebelten Zustand mit freien Radikalen befindet, so dass ein Gemisch bildbar ist, wobei das Gemisch über einen Verteiler (8) in den Luftstrom zurückführbar ist, **dadurch gekennzeichnet, dass**
der Luftkanal (1) der einer Klimaanlage und/oder einer Belüftungseinrichtung ist und der Verteiler (8) für die behandelte Luft im Luftkanal (1) in Strömungsrichtung (11) vor dem ersten Auslass (12) in die zu klimatisierenden und/oder zu belüftenden Räume angeordnet ist.

2. Vorrichtung nach Anspruch 1, **dadurch gekennzeichnet, dass**
das Becken (6) Wasserstoffperoxid in wässriger Lösung (5) als chemische Substanz, bevorzugt mit freien Radikalen, mehr bevorzugt mit OH- und/oder O-Radikalen enthält.

3. Vorrichtung nach Anspruch 2, **dadurch gekennzeichnet, dass**
die Konzentration des Wasserstoffperoxids von 1 bis 30%, bevorzugt 3 bis 25%, mehr bevorzugt von 5 bis 15%, und am meisten bevorzugt 5 bis 8% beträgt.

4. Vorrichtung nach einem der Ansprüche 1 bis 3, **dadurch gekennzeichnet, dass**
die chemische Substanz, bevorzugt mit den freien Radikalen, eine stabilisierende Phosphorsäure enthält, vorzugsweise in einer Konzentration von 0,01 bis 0,5 g in wässriger Lösung, mehr bevorzugt in einer Konzentration von 0,05 bis 0,5 g in wässriger Lösung.

5. Vorrichtung nach einem der Ansprüche 1 bis 4, **dadurch gekennzeichnet, dass**
der Verteiler (8) aus Platin oder einer Platinlegierung besteht.

6. Vorrichtung nach einem der Ansprüche 1 bis 5, **dadurch gekennzeichnet, dass**
der zur Behandlung umgeleitete Luftstrom aus dem Luftkanal (1) ein Teilluftstrom ist, der so bemessen ist, dass die den Verteiler (8) verlassende Luft ausreichend ist, um den vollen Luftstrom ansteckungsresistent zu machen.

7. Vorrichtung nach einem der Ansprüche 1 bis 5, **dadurch gekennzeichnet, dass**
der gesamte Luftstrom eine Behandlung erfährt, wobei die den Verteiler (8) verlassende Luft ansteckungsresistent ist.

8. Vorrichtung nach einem der Ansprüche 1 bis 7, **dadurch gekennzeichnet, dass**
die in den Behandlungsraum abgegebenen Mengen an freie Radikale enthaltenden oder bildenden flüssigen oder gasförmigen chemischen Substanzen steuer- bzw. regelbar ist.

9. Vorrichtung nach einem der Ansprüche 1 bis 8, **dadurch gekennzeichnet, dass**
zum Vernebeln der freie Radikale enthaltenden oder bildenden chemischen Substanzen Piezoelemente (7) eingesetzt sind.

10. Vorrichtung nach einem der Ansprüche 1 bis 9, **dadurch gekennzeichnet, dass**
eine oder mehrere Anschlüsse zum Einströmen von Luft bestehen, die vorzugsweise eine trichterförmige Öffnung entgegen der Strömungsrichtung (12) der Luft aufweisen.

11. Vorrichtung nach einem der Ansprüche 1 bis 10, **dadurch gekennzeichnet, dass**
die Zurückführung der behandelten Luft über den als Rohrgitter im Luftkanal (1) ausgebildeten Verteiler (8) erfolgt, wobei die Rohre des Rohrgitters Öffnungen in Form von Bohrungen bzw. Schlitzen gerichtet in Strömungsrichtung (12) aufweisen.

12. Vorrichtung nach Anspruch 11, **dadurch gekennzeichnet, dass**
die Rohre des Rohrgitters im montierten Zustand senkrecht oder nahezu senkrecht angeordnet sind.

13. Vorrichtung nach einem der Ansprüche 9 bis 10, **dadurch gekennzeichnet, dass**
bei Verneblern auf piezoelektrischer Grundlage dem Verteiler (8) in Strömungsrichtung (11) ein Faraday'scher Käfig (9) nachgeordnet ist.

14. Vorrichtung nach einem der Ansprüche 1 bis 11, **dadurch gekennzeichnet, dass**
alle Mittel zur Luftreinigung und/oder Luftdesinfektion in einer baulichen Einheit eines Gehäuses (2) zusammengeführt sind, wobei aus dem Gehäuse (2) die Luftanschlüsse herausragen und das Gehäuse (2) über Befestigungsmittel für die Montage und Abdichtung gegenüber dem Luftkanal (1) verfügt.

15. Verfahren zur Luftreinigung von in einem Luftkanal (1) strömender Luft von Bakterien und/oder Viren und/oder anderweitigen Keimen und/oder Partikel, wobei mindestens ein Teilluftstrom bypassartig in ein Gehäuse (2) mit einem darin angeordneten Luftbehandlungsraum umgelenkt wird, im Luftbehandlungsraum sich eine chemische Substanz in gasförmigen oder vernebelten Zustand mit freien Radikalen befindet, so dass ein Gemisch gebildet wird in dem die Bakterien und/oder Viren und/oder anderweitigen Keimen und/oder Partikel durch in Kontakt treten mit und/oder Andocken der freien Radikale unschädlich/unwirksam gemacht werden, und die so behandelte Luft in den Luftkanal (1) zurückgeführt und durch einen Verteiler (8) verteilt wird.
